Europäisches Patentamt

European Patent Office

Office européen des brevets

(19)

(11) Veröffentlichungsnummer: **0 193 032**

**A2**

(12) # EUROPÄISCHE PATENTANMELDUNG

(21) Anmeldenummer: **86101814.1**

(22) Anmeldetag: **13.02.86**

(51) Int. Cl.⁴: **A 61 K 37/24**
**C 12 P 21/02, C 12 N 15/00**

(30) Priorität: **13.02.85 DE 3504956**

(43) Veröffentlichungstag der Anmeldung:
**03.09.86 Patentblatt 86/36**

(84) Benannte Vertragsstaaten:
**BE CH DE FR GB IT LI NL SE**

(71) Anmelder: **Gesellschaft für Biotechnologische**
**Forschung mbH (GBF)**
**Mascheroder Weg 1**
**D-3300 Braunschweig-Stöckheim(DE)**

(72) Erfinder: **Mühlradt, Peter, Prof.Dr.**
**Schubertstrasse 5**
**D-3300 Braunschweig(DE)**

(74) Vertreter: **Boeters, Hans Dietrich, Dr. et al,**
**Boeters, Bauer & Partner Thomas-Wimmer-Ring 14**
**D-8000 München 22(DE)**

(54) Zusammensetzung umfassend oder enthaltend Cytokin, Herstellungsverfahren, DNA-Sequenz und deren Verwendung.

(57) Die Erfindung betrifft eine Zusammensetzung, die Cytokin (CEF = Cytotoxicity Enhancing Factor) umfaßt oder enthält, ein Herstellungsverfahren, eine DNA-Sequenz, die das Cytokingen umfaßt, und deren Verwendung.

EP 0 193 032 A2

Croydon Printing Company Ltd.

**BOETERS, BAUER & PARTNER**
PATENTANWÄLTE
EUROPEAN PATENT ATTORNEYS

**0193032**

THOMAS-WIMMER-RING 14
D-8000 MÜNCHEN 22

PAe BOETERS, BAUER & PARTNER
THOMAS-WIMMER-RING 14, D-8000 MÜNCHEN 22

• 1

DIPL.-CHEM. DR. HANS D. BOETERS
DIPL.-ING. ROBERT BAUER
MÜNCHEN

DIPL.-ING. VINCENZ v. RAFFAY
DIPL.-CHEM. DR. THOMAS FLECK
HAMBURG

TELEFON: (089) 22 00 92
TELEFAX: (089) 29 68 13
TELEX: 5 24 878 rrm
TELEGRAMME: PROVENTION, MÜNCHEN

Unser Z.:    4392
Anmelderin: Gesellschaft für Biotechnologische
            Forschung mbH (GBF),
            Mascheroder Weg 1, D-3300 Braunschweig

Zusammensetzung umfassend oder enthaltend Cytokin, Herstellungsverfahren, DNA-Sequenz und deren Verwendung

Die Erfindung betrifft eine Zusammensetzung, die Cytokin
(CEF = Cytotoxicity Enhancing Factor) umfaßt oder enthält,
ein Herstellungsverfahren, eine DNA-Sequenz, die das Cytokin**gen**
umfaßt, und deren Verwendung.

Unter T-Lymphozyten versteht man eine heterogene Population
weißer Blutzellen. Eine Subpopulation, die sogenannten
zytolytischen Vorläuferzellen (CTLP), können zu zytolytischen Effektorzellen (sog. "Killer-T-Zellen"; CTL) ausdifferenzieren; vgl. McDonald & Lees (J. Exp. Med. 150
(1979) 196). McDonald & Lees machen allerdings keine Angaben
darüber, ob ein Cytokin für das Ausdifferenzieren verantwortlich ist. Aus dem im folgenden noch angeführten Stand der
Technik darf man schließen, daß dieses Ausdifferenzieren in
Gegenwart reifer T-Zellen erfolgt. CTL dienen als Bestandteile des Immunsystems dazu, von Viren befallene Zellen und
Tumorzellen zu eliminieren.

Auf dem Veterinärsektor nimmt man an, daß bestimmte Aktivitäten in Kulturüberständen von Nager-Milzzellen oder

-Zellinien CTLP zu CTL ausdifferenzieren lassen können; vgl. Raulet & Bevan (Nature 296 (1982) 754) und Taku et al. (J. Immunol. 133 (1984) 502). Nach diesen beiden Arbeiten sind T-Lymphozyten, d.h. von ihnen gebildete Lymphokine Quelle dieser Aktivität. Nach Taku et al. sollen allerdings auch zahlreiche andere Kulturmedien, u.a. Kulturmedien der murinen myelo-monozytären Zellinie WEHI-3, eine Aktivität aufweisen, die (schließlich) zum Ausdifferenzieren von CTLP führt. Der Aktivitätsbefund für WEHI-3-Kulturmedien war nach diesem Stand der Technik folgendermaßen zu erklären. In diesen Kulturmedien tritt bekanntermaßen Interleukin-3 (IL-3) als Lymphokin auf; vgl. Garland in Pick: Lymphokines, Band 9 (1984) 153 bis 200, insbesondere 164 bis 165 und 200. IL-3 ist kein Wachstumsfaktor für T-Zellen. Von diesem Faktor ist nicht bekannt, daß er zur Bildung von CTL ("Killer-T-Zellen") führen kann. Er kann jedoch als Differenzierungsfaktor auf frühe T-Lymphozyten (nicht zu verwechseln mit CTLP!) wirken. Diese können ihrerseits im Verlauf der 5-tägigen Kultur-, Wachstums- bzw. Reifeperiode das Lymphokin bilden, das zur Ausdifferenzierung von CTLP zu CTL führt.

Erfindungsgemäß wurde nun auf dem Human-Sektor eine Cytokin-Aktivität, die CTLP zu Killer-T-Zellen ausreifen läßt, überraschenderweise auch dann festgestellt, wenn man das Kulturmedium bzw. den Kulturüberstand myelo-monozytärer Human-Zellinien auf nicht proliferierende CTLP einwirken läßt. Diese Cytokin-Aktivität ist dadurch gekennzeichnet, daß sie CTLP zu Killer-T-Zellen ausreifen läßt. Daraus folgt, daß dieses Cytokin zumindest im Human-Bereich unmittelbar von myelo-monozytären Human-Zelllinien gebildet wird.

Gegenstand der Erfindung ist eine Zusammensetzung mit einem Gehalt an Cytokin (CEF) aus myelo-monozytären Human-Zelllinien. Diese Zusammensetzung kann zur Behandlung von Immundefizienz verwendet werden.

Gegenstand der Erfindung ist ferner ein Verfahren zur Herstellung der genannten Zusammensetzung, bei dem man myelomonozytäre Human-Zellinien kultiviert, den Kulturüberstand gewinnt und gegebenenfalls das Cytokin durch übliche Methoden der Protein-Chemie anreichert. Als übliche Anreicherungsmethoden bieten sich eine Ammoniumsulfatfällung und/oder die Säulenchromatographie an.

Myelo-monozytäre Zellinien sind im Handel erhältlich. Beispiele für brauchbare myelo-monozytäre Human-Zellinien sind HL 60 (ATCC CCL240) und U937 (ATCC CRL1593).

Nachstehend wird die Erfindung durch Beispiele näher erläutert.

Beispiel 1

Es werden HL-60-Zellen in RPMI-1640-Medium mit 5-proz. fötalem Kälberserum bis zu einer Dichte von etwa $1 \times 10^6$ Zellen/ml gezüchtet. Das Kulturmedium wird durch niedrigtourige Zentrifugation geerntet. Das CEF enthaltende Kulturmedium bewirkt bei einer Verdünnung von 1:10 eine Erhöhung der spezifischen lytischen Aktivität von etwa 30 % gegenüber einer Mediumkontrolle in den folgenden Testsystemen:

(1) Es werden $3 \times 10^5$ Milzleukozyten einer CBA/J-Maus in 0,2 ml Mikrotiterplattenkulturen in Gegenwart von

    (a) 2,5 /ug ConA/ml,
    (b) 0,25 /ug Mitomycin C/ml und
    (c) variablen Volumina eines Kulturüberstandes der HL-60-Zellen 40 bis 48 h in RPMI-1640-Medium, 5-proz. fötalem Kälberserum und $2,5 \times 10^{-5}$ m 2-Merkaptoethanol unter den üblichen Bedingungen kultiviert. Am Ende der Kulturperiode wird die lytische Aktivität der Milzleukozyten durch Zugabe von $10^4$ $^{51}$Cr-markierten P815-
Tumorzellen in Gegenwart von 2,5 ,ug/ml Phytohämagglutinin im üblichen 4-Stunden-$^{51}$Cr-Release-Test gemessen.

(2) Es werden 6 x $10^5$ Thymozyten einer CBA/J-Maus in 0,2 ml
Mikrotiterplattenkulturen in Gegenwart von

(a) 4 ,ug ConA/ml und

(b) variablen Volumina eines Kulturüberstandes der
HL-60-Zellen etwa 72 h in RPMI-1640-Medium mit 5-proz.
fötalem Kälberserum und 2,5 x $10^{-5}$ m 2-Merkaptoethanol
kultiviert. Am Ende der Kulturperiode wird die zytolytische Aktivität der Thymozyten wie vorstehend angegeben
gemessen.

Beispiel 2

Zur Gewinnung und Überführung des Gens des erfindungsgemäßen
Cytokins im Hinblick auf seine Expression befolgt man den
experimentellen Text von DE-A-3 203 537.3 (vgl. auch
Lindenmaier et al. in Nucleic Acids Research, 10 (1982)
1243) unter Verwendung von L$^+$k$^-$-Zellen und E.-coli-HB101 als
eukaryontische bzw. prokaryontische Wirtszellen.

BOETERS, BAUER & PARTNER
PATENTANWÄLTE
EUROPEAN PATENT ATTORNEYS

THOMAS-WIMMER-RING 14
D-8000 MÜNCHEN 22

PA: BOETERS, BAUER & PARTNER
THOMAS-WIMMER-RING 14, D-8000 MÜNCHEN 22

DIPL.-CHEM. DR. HANS D. BOETERS
DIPL.-ING. ROBERT BAUER
MÜNCHEN

DIPL.-ING. VINCENZ v. RAFFAY
DIPL.-CHEM. DR. THOMAS FLECK
HAMBURG

TELEFON: (089) 22 00 92
TELEFAX: (089) 29 66 13
TELEX: 5 24 878 rrm
TELEGRAMME: PROVENTION, MÜNCHEN

0193032

## Patentansprüche

1. Zusammensetzung umfassend oder enthaltend Cytokin (CEF) aus myelo-monozytären Human-Zellinien.

2. Verfahren zur Herstellung einer Zusammensetzung gemäß Anspruch 1, dadurch g e k e n n z e i c h n e t , daß man myelo-monozytäre Human-Zellinien kultiviert, den Kulturüberstand gewinnt und gegebenenfalls das Cytokin (CEF) durch übliche Methoden der Protein-Chemie anreichert.

3. Verfahren nach Anspruch 2, dadurch g e k e n n - z e i c h n e t , daß man eine myelo-monozytäre Human-Zellinie kultiviert, die kein Interleukin-2 (IL-2) produziert.

4. Verfahren nach Anspruch 2, dadurch g e k e n n - z e i c h n e t , daß man das Cytokin (CEF) durch Ammoniumsulfatfällung und/oder Säulenchromatographie anreichert.

5. DNA-Sequenz umfassend das Gen des Cytokins (CEF) gemäß Anspruch 1 und gewinnbar aus myelo-mono-zytären Human-Zellinien.

6. Verwendung der DNA-Sequenz gemäß Anspruch 5 zur mikrobiologischen Herstellung der Zusammensetzung gemäß Anspruch 1.